# EUROPEAN PATENT SPECIFICATION

(11) **EP 3 258 942 B1**
(45) Date of publication and mention of the grant of the patent: **13.01.2021**
(21) Application number: 16752852.0
(22) Date of filing: 12.02.2016
(51) Int. Cl.: A61K 31/05, A61K 31/74, A61K 31/34, A61K 31/194, A61K 31/355, A61K 31/4166, A61K 31/4375, A61K 31/7048, A61K 45/06, A61K 8/34, A61K 8/92, A61K 9/00, A61K 36/185, A61Q 3/00, A61Q 5/00, A61Q 15/00, A61Q 19/00, A61Q 19/08, A61Q 19/10, A61P 17/00

(54) **COSMETIC AND TOPICAL COMPOSITIONS COMPRISING CANNABIGEROL AND CANNABIDIOL**
KOSMETISCHE UND TOPISCHE ZUSAMMENSETZUNG ENTHALTEND CANNABIGEROL UND CANNABIDIOL
COMPOSITIONS COSMÉTIQUES ET TOPIQUES COMPRENANT DU CANNABIGÉROL ET DU CANNABIDIOL

(30) Priority: 16.02.2015 US 201562116905 P
(43) Date of publication of application: 27.12.2017
(73) Proprietor: APIRX Pharmaceutical USA, LLC, New York NY 10022 (US)
(72) Inventor: CHANGOER, Lekhram, 2987 VD Ridderkerk (NL); ANASTASSOV, George, New York, NY 10022 (US)
(74) Representative: Nederlandsch Octrooibureau
(86) International application number: PCT/US2016/017814
(87) International publication number: WO 2016/133824

(56) References cited:
- WO-A1-2006/015675
- WO-A1-2008/079898
- WO-A2-2008/024408
- FR-A1- 2 966 698
- US-A1- 2003 068 347
- US-A1- 2004 120 909
- US-A1- 2004 120 909
- US-A1- 2007 280 898
- US-A1- 2008 286 390
- US-A1- 2010 166 891
- US-A1- 2010 166 891
- US-A1- 2011 027 327
- US-A1- 2012 237 556
- US-A1- 2013 058 885
- US-A1- 2013 142 881
- US-A1- 2013 319 889
- US-A1- 2014 030 202
- US-A1- 2014 127 148
- DATABASE WPI Week 200963 Thomson Scientific, London, GB; AN 2009-H36887 XP002784997, & KR 2009 0005479 A (JUNG J I) 14 January 2009 (2009-01-14)
- WILKINSON ET AL: "Cannabinoids inhibit human keratinocyte proliferation through a non-CB1/CB2 mechanism and have a potential therapeutic value in the treatment of psoriasis", JOURNAL OF DERMATOLOGICAL SCI, ELSEVIER, AMSTERDAM, NL, vol. 45, no. 2, 19 January 2007 (2007-01-19), pages 87-92, XP005836247, ISSN: 0923-1811, DOI: 10.1016/J.JDERMSCI.2006.10.009
- MARIANGELA PUCCI ET AL: "Epigenetic control of skin differentiation genes by phytocannabinoids : Epigenesis of skin genes by phytocannabinoids", BRITISH JOURNAL OF PHARMACOLOGY, vol. 170, no. 3, 17 September 2013 (2013-09-17), pages 581-591, XP055508939, UK ISSN: 0007-1188, DOI: 10.1111/bph.12309
- LAYTON C. ET AL.: "Analysis of cannabinoids in hemp seed oils by HPLC using PDA detection", LIQUID CHROMATOGRAPHY APPLICATION NOTED, 2015, pages 1-6,
- PACIFICO D ET AL: "Time course of cannabinoid accumulation and chemotype development during the growth of Cannabis sativa L", EUPHYTICA, KLUWER ACADEMIC PUBLISHERS, DO, vol. 160, no. 2, 18 August 2007 (2007-08-18), pages 231-240, XP019572552, ISSN: 1573-5060
- DE MEIJER E P M ET AL: "The inheritance of chemical phenotype in Cannabis sativa L. (II): Cannabigerol predominant plants", EUPHYTICA, KLUWER ACADEMIC PUBLISHERS, DO, vol. 145, no. 1-2, 1 September 2005 (2005-09-01), pages 189-198, XP019240971, ISSN: 1573-5060, DOI: 10.1007/S10681-005-1164-8
- Molleken H. et al.: "Cannabinoids in seed extracts of Cannabis sativa cultivars", , 1997, pages 1-12, Retrieved from the Internet: URL:https://www.druglibrary.net/olsen/HEMP /IHA/jiha4201.html [retrieved on 2019-10-24]
- GIOVANNI APPENDINO ET AL: "Antibacterial cannabinoids from Cannabis sativa: a structure-activity study", JOURNAL OF NATURAL PRODUCTS, AMERICAN CHEMICAL SOCIETY, US , vol. 71, no. 8 1 August 2008 (2008-08-01), pages 1427-1430, XP002679920, ISSN: 0163-3864, DOI: 10.1021/NP8002673 Retrieved from the Internet: URL:http://pubs.acs.org/doi/abs/10.1021/np 8002673 [retrieved on 2008-08-06]
- 14 Beauty products made with hemp - Elle XP055713057
- James Geiwitz ET AL: "THC in Hemp Foods and Cosmetics: The Appropriate Risk Assessment", , 15 January 2001 (2001-01-15), pages 1-35, XP055713063, Retrieved from the Internet: URL:http://citeseerx.ist.psu.edu/viewdoc/d ownload?doi=10.1.1.173.4786&rep=rep1&type= pdf [retrieved on 2020-07-08]
- VOGL C R ET AL: "Hemp (Cannabis sativa L.) as a Resource for Green Cosmetics: Yield of Seed and Fatty Acid Compositions of 20 Varieties Under the Growing Conditions of Organic Farming in Austria", JOURNAL OF INDUSTRIAL HEMP,, vol. 9, no. 1, 1 January 2004 (2004-01-01) , pages 51-68, XP002536675, ISSN: 1537-7881, DOI: 10.1300/J237V09N01_06
- CINZIA CITTI ET AL: "Cannabinoid Profiling of Hemp Seed Oil by Liquid Chromatography Coupled to High-Resolution Mass Spectrometry", FRONTIERS IN PLANT SCIENCE, vol. 10, 13 February 2019 (2019-02-13), XP055713064, DOI: 10.3389/fpls.2019.00120

## Description

### BACKGROUND OF THE INVENTION

### Field of the Invention

This invention relates to topical cosmetics and dermatological compositions and the topical application of such compositions to the skin for the prevention and/or treatment of damage to the skin. The topical compositions for dermal application comprise hemp oil extractable from the *Cannabis sativa* plant containing cannabigerol, a non-psychoactive phytocannabinoid, and cannabigerol, at least one anti-oxidant, at least one anti-microbial agent, at least one anti-inflammatory agent, at least one preservative, at least one emulsifier, suitable cosmetic additives and carriers, and de-ionised water, as defined in the claims. The topical composition is anti-aging day cream, anti-aging night cream, eye cream, hand and nail cream, lip balm, acne cream and tonic, face wash, shampoo and conditioner, body wash, deodorant, or anti-diaper rash cream.

### Description of the Related Technology

The cannabis plant has many naturally occurring substances that are of great interest in the fields of science and medicine. Isolated compounds from the cannabis plant include Δ⁹-tetrahydrocannabinol (THC), cannabidiol (CBD), cannabichromene (CBC), cannabigerol (CBG), cannabinol (CBN), cannabidivarin (CBDV), among other compounds. While THC has psychoactive effects, CBD, CBC, CBG, and CBDV do not. Isolated compounds from the cannabis plant are called cannabinoids. There are a total of eighty-five (85) cannabinoids that have been isolated from the cannabis plant. Cannabinoids have been investigated for possible treatment of seizures, nausea, vomiting, lack of appetite, pain, arthritis, inflammation, and other conditions.

The IUPAC nomenclature of THC is (-)-(6aR,10aR)-6,6,9-trimethyl-3-pentyl-6a,7,8,10a-tetrahydro-6H-benzo[c]chromen-1-ol. CBD's IUPAC nomenclature is 2-((1S,6S)-3-methyl-6-(prop-1-en-2-yl)cyclo-hex-2-enyl)-5-pentylbenzene-1,3-diol). CBC has the IUPAC nomenclature of 2-methyl-2-(4-methylpent-3-enyl)-7pentyl-5-chromenol. CBG has the IUPAC nomenclature of 2-[(2E)-3,7-dimethylocta-2,6-dienyl]-5-pentyl-benzene-1,3-diol. These are among the most prominent compounds in the family of compounds extracted from the cannabis plant referred to as cannabinoids.

Because it is a relatively unknown cannabinoid, cannabigerol (CBG) remains understudied and its effects are only just starting to become clear. CBG is a non-psychoactive cannabinoid found in the cannabis plant. All cannabinoids in the early stage of the cannabis plant's life begins as CBG. CBG is found in higher concentrations in hemp plants as opposed to marijuana plants that are grown to have higher concentrations of tetrahydrocannabinol (THC). CBG has been found to act as a high affinity α₂-adrenergic receptor agonist, a moderate affinity 5-HT_{1A} receptor antagonist, and a low affinity CB₁ receptor antagonist. It binds with the CB2 receptor, but it is currently unknown whether it acts as an agonist or antagonist.

CBG has been shown to relieve intraocular pressure, which may help in the treatment of glaucoma. It can also be used to treat inflammatory bowel disease. CBG also inhibits the uptake of γ-aminobutyric acid (GABA) in the brain, which in turn helps decrease anxiety. Research on rats has shown that CBG has an anti-nausea and anti-emetic effect, although human testing has yet to be carried out. One study even suggests that CBG inhibits the growth of color cancer cells. Another study found that CBG could potentially be helpful to treat many central nervous system disorders such as epilepsy.

Cannabinoids can be isolated by extraction or cold pressing from cannabis plants. Plants in the cannabis genus include *Cannabis sativa, Cannabis ruderalis,* and *Cannabis indica.* These plants are the natural sources of cannabinoids. Cannabinoids are also available in synthetic forms. Methods to synthesize cannabinoids in lab settings were discovered and are still currently practiced. Synthetic cannabinoids are more targeted, in that the synthetic compound usually comes isolated without other cannabinoids mixed in.

Nabilone (racemic(6aR,10aR)-1-hydroxy-6,6-dimethyl-3-(2-methyloctan-2-yl)-7,8,10,10a-tetrahydro-6H-benzo[c]chromen-9(6aH)-one), a synthetic cannabinoid, is believed to have fewer undesired side effects than THC. Nabilone mimics the chemical compound structure of THC. THC also exists in synthetic form under the name Dronabinol ((-)-(6aR,10aR)-6,6,9-trimythel-3-pentyl-6a,7,8,10a-tetrahydro-6H-benzo[c]chromen-1-ol)). These synthetic cannabinoids are sold for food supplement purposes and are being investigated for medicinal purposes. The U.S. Food and Drug Administration approved nabilone for treatment of chemotherapy-induced nausea and vomiting. In the United States, nabilone is marketed under the name Cesamet®.

Cosmetics are body-care substances used to enhance the human body's appearance or odor. Cosmetics are used to cleanse, beautify, promote attractiveness, and alter appearance without affecting the body's structure or function.

Various types of cosmetics exist. Generally, they are cosmetics applied to the skin (face and body), cosmetics applied to the lips, cleansing cosmetics (soap, shampoo, and conditioner), cosmetics applied to change odor (perfume, body mist, deodorant), and cosmetics applied to the eyes (mascara, eyebrow pencil).

Polyunsaturated fatty acids (omega-6 and omega-3) are essential to skin health. Metabolism of linoleic acid and alpha-linoleic acid is limited in the skin. Topical and oral supplementations of essential fatty acids are effective means to supply these essential fatty acids to the skin. Some anti-aging cosmetic products do not include these important polyunsaturated fatty acids.

Essential fatty acids (EFAs) found in hemp oil act as moisturizers to trap moisture on the skin. Moreover, the fatty acids ratio in hemp oil closely matches the human skin composition and body, which is 3:1 ratio of omega-6 to omega-3 essential fatty acids. EFAs are proven to play a preventive role in skin aging and a healthy moisture balance for the skin.

Omega fatty acids are a group of unsaturated fatty acids considered to be important to physiological development in mammals. In particular, omega-3 fatty acids are considered to be vital for normal metabolism. These fatty acids receive their names based on the location of the first double bond from the tail end (omega end) of the carbon chain.

Omega-3 fatty acids are a group of three (3) fatty acids called α-linolenic acid (ALA), eicosapentaenoic acid (EPA), and docosahexaenoic acid (DHA). Mammals have limited ability to synthesize omega-3 fatty acids. Given their importance in metabolism, mammals must consume omega-3 fatty acids to ensure normal metabolism.

Omega-6 fatty acids are a family of unsaturated fatty acids that have in common a carbon-carbon double bond in the n-6 position. Omega-6 fatty acids are considered to be important to a mammal's diet. The relative ratio of omega-6 to omega-3 fatty acids in Western diet is about 10:1 and up to 30:1. An excessive ratio of omega-6 to omega-3 fatty acids may interfere with omega-3 activity, storage, and conversion to eicosanoid precursors.

Terpenes refer to a large class of organic compounds produced by a variety of plants, particularly conifers. It is believed that they protect the plants that produce them by deterring herbivores and actually attracting predators and parasites of herbivores. Terpenes are the major ingredient in resin and form some of the major biosynthetic building blocks within nearly every living creature. Terpenes are the primary constituents of the essential oils of many plants. These essential oils are widely used as natural flavor additives for food, as fragrances in perfumes, and in medicine and alternative medicines in the form of aromatherapy. They are released by trees more actively in warmer weather and act as a natural form of cloud seeding, helping to regulate the temperature of the forest. Terpenes form the aroma and flavor in hops, which are highly desirable in some beers.

A range of terpenes have been identified as high-value chemicals in food, cosmetic, pharmaceutical, and biotechnology industries. They are generally difficult to produce synthetically, due to their complex structures. Research has shown that many terpenes possess qualities that make them ideal active ingredients as part of natural agricultural pesticides. Terpin hydrate is a derivative of turpentine, and as an expectorant or humectant it is commonly used in the treatment of chronic or acute bronchitis and related conditions.

Cosmetics applied to the skin, whether stay-on or rinse-off, usually have anti-microbial, anti-oxidant, anti-aging, and wound healing properties. Various substances are added to cosmetics to achieve these effects, and continuous efforts are still made to identify substances with these properties to be used in cosmetics.

US-A-2013/319889 discloses a topical composition for application to the lips or the skin comprising hemp (seed) oil, which restores damaged skin. US-A-2007/280898 discloses a topical dermatological composition for the treatment of skin blemishes, e.g. scars and stretch marks. Example 1 describes a cream comprising hemp seed oil. US-A-2008/286390 discloses a skin care composition, e.g. a cream to treat eczema, comprising hemp (seed) oil. KR-A-20090005479 describes a hair cream with an oil system comprising hemp seed oil. US-A-2013/058885 discloses a shampoo with a shampoo base comprising hemp oil. US-A-2010/166891 discloses a composition for the topical treatment of human skin, preferably in the form of a cream, comprising hemp (seed) oil. US-A-2004/120909 discloses an antiperspirant composition containing natural oil, which can be hemp oil. FR-A-2 966 698 discloses that a mixture of 35-45% cannabigerol, 3-10% cannabidiol and 0.2-2% carmagerol has an antimicrobial and antibacterial effect and can be used in a dermo-cosmetic topical composition for the treatment of skin infections in animals.

### SUMMARY

The present invention relates to topical compositions comprising hemp oil extractable from the *Cannabis sativa* plant containing cannabigerol at 3% to 24% by weight of the oil and cannabidiol at 1% to 10% by weight, ; as defined in the claims. Preferably, the oil comprises 0.2% to 10% by weight of the topical leave-on composition. In the rinse off formulations, the concentrations of CBG hemp oil or cannabis oil are at 0.05% to 1.0% by weight of the topical rinse-off composition. The topical composition is formulated to be anti-aging day cream, anti-aging night cream, eye cream, hand and nail cream, lip balm, acne cream and tonic, diaper rash cream, shampoo or conditioner, deodorant spray, face wash, or body wash. The present invention also relates to a topical composition comprising hemp oil comprising cannabigerol and cannabidiol as defined in the claims for use in the therapeutic treatment of skin conditions, preferably acne. The present disclosure also relates to methods to make the topical composition, and methods to use the topical composition for anti-aging, nourishing, and cleansing purposes.

### ABBREVIATIONS

CBC: Cannabichromene
CBD: Cannabidiol
CBDV: Cannabidivarin
CBG: Cannabigerol
CBN: Cannabinol
GABA: γ-aminobutyric acid
INCI: International Cosmetic Ingredient Nomenclature Committee
IUPAC: International Union of Pure and Applied Chemistry
SPF: Sun Protection Factor
THC: Tetrahydrocannabinol
UV: Ultraviolet

### DETAILED DESCRIPTION OF CERTAIN INVENTIVE EMBODIMENTS

The invention is defined by the claims. Any subject-matter falling outside the scope of the claims is provided for information purposes only. This present invention is capable of being embodied in various forms. The description below of several embodiments is made with the understanding that the present disclosure is to be considered as an exemplification of the claimed subject matter, and is not intended to limit the attached claims to the specific embodiments illustrated. The headings used throughout this disclosure are provided for convenience only and are not to be construed to limit the claims in any way. Embodiments illustrated under any heading may be combined with embodiments illustrated under any other heading.

As used herein, the verb "to comprise" in this description, claims, and other conjugations are used in their non-limiting sense to mean those items following the word are included, but items not specifically mentioned are not excluded.

Reference to an element by the indefinite article "a" or "an" does not exclude the possibility that more than one of the elements are present, unless the context clearly requires that there is one and only one of the elements. The indefinite article "a" or "an" thus usually means "at least one." Additionally, the words "a" and "an" when used in the present document in concert with the words "comprising" or "containing" denote "one or more."

The word "cannabinoid" used in this description is used to mean any compound that interacts with a cannabinoid receptor and other cannabinoid mimetics, including, but not limited to, certain tetrahydropyran analogs (Δ⁹-tetrahydrocannabinol, Δ⁸-tetrahydrocannabinol, 6,6,9-trimythel-3-pentyl-6H-dibenzo[b,d]pyran-1-ol, 3-(1,1-dimethylheptyl)-6,6a7,8,10,10a-hexahydro-1-1hydroxy-6,6-dimythel-9H-dibezo[b,d]pyran-9-ol,(-)-(3S,4S)-7-hydroxy-delta-6-tetrahydrocannabinol-1,1-dimethylheptyl, (+)-(3S,4S)-7-hydroxy-Δ-6-tetrahydrocannabinol, and Δ⁸-tetrahydrocannabinol-11-oic acid); certain piperidine analogs (e.g., (-)-(6S,6aR,9R,10aR)-5,6,6a,7,8,9,10,10a-octahydro-6-methyl-1-3-[(R)-1-methyl-4-phenylbutoxyl-1,9-phenanthridinediol 1-acetate)); certain aminoalkylindole analogs (e.g., (R)-(+)-[2,3-dihydro-5-methyl-3-(4-morpholinylm-ethyl)-pyrrolo[1,2,3,-de]-1,4-benzoxazin-6-yl]-1-naphthelenyl-methanone); certain open pyran-ring analogs (e.g., 2-[3-methyl-6-(1-methylethenyl-2-cyclohexen-1-yl]-5-pentyl-1, 3-benzendi-ol, and 4-(1,1-dimethylheptyl)-2,3'-dihydroxy-6'-α-(3-hydroxypropyl)-1',-2',3',4',5',6'-hexahydrobiphenyl), their salts, solvates, metabolites, and metabolic precursors.

As used in this application, cannabidiol is obtained from industrial hemp extract with a trace amount of THC or from cannabis extract using high-CBD cannabis cultivars.

As used in this application, cannabigerol is obtained from industrial hemp extract with a trace amount of THC or from cannabis extract.

As used in this application, cannabinol is obtained from cannabis extract or from industrial hemp extract.

Cannabinoids derived from natural sources usually come in oily form, known as cannabis oil, hashish oil, or hemp oil, depending on its origin, and is extract of the plant *Cannabis sativa* L.. *Cannabis sativa* L.'s seed oil with naturally occurring cannabinoid content may also be used. The oil has a liquid to paste-like profile at room temperature, and is hydrophobic. According to the disclosure, *Cannabis sativa* L.'s seed oil or plant oil high in naturally occurring CBG may be used.

The topical dermatological compositions in these embodiments comprise hemp oil extractable from the *Cannabis sativa* plant containing cannabigerol (CBG) at 3% to 24% by weight. Also present is cannabidiol (CBD) at 3% to 10% by weight, and optionally THC, CBC, CBN, or CBDV at lower weight percentage.

Cannabis oil or hemp oil with high naturally occurring CBG has anti-bacterial, anti-aging, neuro-protective, moisturizing, and anti-oxidant properties. When used for dermal application, CBG oil may prevent and treat damage on skin, and may moisturize the skin to give a youthful appearance. Anti-bacterial properties may help minimize the chance of acne development, as well as treating on-going acne on the dermis.

Topical dermal compositions used on the skin during daylight should have ultraviolet (UV) ray protection properties to protect the dermis from sun damage. Depending on the strength of sunlight and geographic areas, the demand for UV ray protection may vary. CBG has neuro-protective properties as well as anti-oxidant properties, which may be useful is prevention of photodamage of the skin from UV rays exposures.

Terpenes in hemp oil or cannabis oil may provide anti-microbial properties, which may be desirable in cosmetics used for cleansing and/or in cosmetics applied to the skin. Terpenes may also add to the flavor of the cosmetics as a whole.

In embodiments, the topical composition may contain hemp oil with naturally occurring CBG and CBD at 0.2% to 10% by weight of the total composition, more preferably at 2% to 5% by weight of the total composition, for stay-on formulations. Stay-on formulations are topical compositions applied to the skin and are not rinsed-off within a few minutes.

For rinse-off formulation, the topical composition may contain hemp oil with naturally occurring CBG and CBD at 0.05% to 1% by weight of the total composition. Rinse-off formulations are topical compositions that may be rinsed off with water within a few minutes after application.

Depending on the specific formulation, the topical composition may comprise other ingredients. Anti-glycation agents may be added to this topical composition. Anti-glycation agents are compounds for preventing and/or reducing the glycation of skin protein, such as a dermal protein like collagen. The anti-glycation agent may inhibit the formation of advanced glycation end products. Anti-glycation agents may be present in the topical composition at 0.1% to 5% by weight of the total composition.

Examples of anti-glycation agents may be extracts of *Vaccinium angustifolium* (blueberry), ergothioneine and its derivatives, hydroxystilbenes and their derivatives, benfotimaine, pyrisoxamine, G-rutin, pyridoxal phosphate, green tea extract, curcumin extract, *Lupius albas* seed oil, or *Kigelia africana* extract. Other anti-glycation agents may also be used.

Anti-inflammatory agents as defined in the claims are added to the topical composition to prevent and treat skin inflammation. Free radicals produced in aging skin may also induce inflammation of the skin and reduce skin immunity. Anti-inflammatory agents in topical compositions may work to reduce inflammation and increase skin health. Useful anti-inflammatory agents may be NSAIDS and COX-2 inhibitors, such as curcumin extract, evodia extract, *Camellia sinesis* extract, cocoa seed butter, citric acid, salicylic acid, tocopherol, loganin, palmatine, *Vitis vinifera* extract, among other anti-inflammatory ingredients. Anti-inflammatory agents may be present in the topical composition at 0.5% to 20% by weight.

Reactive oxygen species (ROS) are either free radicals or non-radical with high oxidative properties. Living tissues have a control mechanism to keep ROS in balance. Endogenous antioxidants defend the human body against ROS. When insufficient endogenous antioxidants are present, antioxidant supplements may work to inhibit the production of ROS by direct scavenging. Antioxidants may also decrease the amount of oxidants in and around our cells, prevent ROS from reaching their biological targets, limit the propagation of oxidants, thwart oxidative stress, and ultimately prevent the aging process.

Anti-oxidants may be useful in cosmetic formulations to ease oxidative stress and prevent collagen degradation in the skin. Anti-oxidants according to the invention for this topical formulation are selected from *Vitis vinifera* extract, tocopherols, 3-carotene, certain anthocyans (blueberry extract, basil grape extract), certain catechins (green tea extract, grape seed extract, litchi extract), certain quercetins (blueberry extract, grape seed extract, apple extract), certain genistein extract (gingoko biloba extract, soja extract), acetyl hexapeptide-*3, Silybum marianum* fruit extract, or silymarin (milk thistle). Anti-oxidants may be incorporated into the topical formulation at 0.1% to 30% by weight of the total composition.

Anti-microbial ingredients protect against the growth of microorganisms in personal care products. Anti-microbial agents may also kill microorganisms present in the cosmetic product from the manufacturing process. Anti-microbial agents may be present in these embodiments to safeguard against microbial overgrowth, which may compromise the composition's quality as a whole.

Antimicrobial agents according to the invention are selected from imidazolidinyl urea, sodium chloride, or certain extracts having anti-microbial activities such as *Matricaria chamomilla* extract, *Aloe vera* extract, *Calendula officinalis* extract, *Kigelia africana* extract, *Lavandulla officinallis* essential oil, *Melaleuca alternifolia* essential oil, or *Cinnamomum zeylanicum* essential oil, or pirocotone olamine. Anti-microbial agents may be present in the topical composition at 0.5% to 15% by weight of the total composition.

UV exposure may lead to multiple cellular damage by generating O2, H₂O₂, [-OH] and other reactive oxygen species. The presence of oxidative groups may lead to skin aging, actinic damage, collagen and elastin degradation. UVB rays (290 - 320 nm) are absorbed by epidermal chromophores such as melanin and urocanic acid and lead to direct molecular damage. UVA rays (320 - 400 nm) penetrate more deeply in the dermis, increase the production of ROS, and contribute to long-term actinic damage. UV ray damages may be mitigated with the use of certain agents that prevent and/or disrupt the destructive reactions caused by UV rays.

Suitable UV ray filter for this topical composition may be butylene glycol dicaprylyate or dicaprate, ethylhexyl methoxycinnamate, bis-ethylhexylloxyphenol methoxyphenyl triazine, camphor benzalkonium methosulfate, diethylhexyl butamido triazone, bisdisulizole disodium, drometrizole trisiloxane, ethylhexyl triazone, methyl anthranilate, 4-methylbenzylidene camphor, methylene bis-benzotriazolyl tetramethylbutyl phenol, octocrylene, butyl methoxydibenzoylmethane, phenylbenzimidazole sulfonic acid, polyacrylamidomethyl benzylidene camphor, or terephthalylidene dicamphor sulfonic acid.

UV ray filters may be added into the topical composition in embodiments used during the day, when skin exposure to sunlight is expected. UV ray filters may not be necessary for the topical compositions used during the night. UV ray filter agents may be present in the topical composition at 2% to 30% by weight of the total composition.

The topical composition may further comprise multiple skin conditioning agents. Skin conditioning agents may work to increase moisture retention of the upper dermis layer, and thus give the skin an appearance of suppleness. Certain skin conditioning agents may work to reduce flaky appearance and attract moisture from the environment, like a humectant.

Suitable skin conditioning agents may be butyrospermum parkii (shea butter), pentylene glycol, acetyl hexapeptide-3, lipobelle soyaglycone, xylitylglucoside, anhydroxyxylitol, xylitol, ethylhexylglycerin, squalene, *Triticum vulgare* germ oil unsaponifiables, ceramide-3, ethylhexyl stearate, biosaccharide gum-1, paraffinum liquidum, isopropyl palminate, glycerin stearate, D-panthenol, lactic acid, tocopherol and tocopherol derivatives, cetearyl alcohol, dicaprylyl carbonate, butylene alcohol, butylene glycol, and carnosine, butylene glycol cocoate, propylheptyl caprylate, glycine soja sterols (soybean extracts), decyl oleate, PPG-26-buteth-26, potassium ascorbyl tocopheryl phosphate, guar hydroxypropyltrimonium chloride, methyl gluceth-10, or avocado oil. Skin conditioning agents may be incorporated into this composition at 0.01% to 30% by weight of the total composition.

In embodiments, the topical composition may be a rash cream to treat diaper rash and other common rashes. In this embodiment, the topical composition may further comprise a soothing agent such as oxidized corn oil to provide a soothing feeling to the skin.

In topical compositions for use on nails or hair, agents with keratolytic effect may be added into the topical composition. Hydrolyzed keratin and/or allantoin with keratolytic effect may be used in these embodiments. Keratolytic agents may be incorporated into this composition at 0.1% to 5% by weight of the total composition.

Additional preservatives are added into the topical composition to extend shelf life and prevent microbial growth. Suitable preservatives may be pentylene glycol, phenoxyethanol, methyl and propylparaben, or ethylhexyl glycerin, or benzyl alcohol. Other preservatives suitable for cosmetic use may also be used. Preservatives may be present in the topical composition at 0.5% to 10% by weight of the total composition.

The topical formulation comprises de-ionized water, as the base, and bulking agent in this formulation. Water may comprise about 40% to about 80% by weight of the topical composition. However, in an embodiment wherein the topical composition is a lip balm, the composition comprises no water (not claimed).

Viscosity controlling agents and emulsifiers may be necessary to bind water-phase ingredients and oil-phase ingredients. Suitable emulsifiers for these embodiments may be hydrogenated lecithin, glycerin, sodium gluconate, acrylates, C10-30 alkyl acrylate crosspolymer, sodium carboxymethyl betaglucan, hydrogenated castor oil, polyglyceryl-3 methylglucose distearate, cetearyl alcohol, behenyl alcohol, butylene glycol, propylene glycol, xanthan gum, potassium cetyl phosphate, polyglyceryl-6 distearate jojoba esters, polyglyceryl-3 beewax, cetyl alcohol, PEG-800, laureth-7, C13-14 isoparrafin, polyisobutene, sodium carboxymethyl betaglucan, PEG-200 hydrogenated glyceryl palmate, cellulose gum, PEG-7 glyceryl cocoate, or aluminum starch octenylsuccinate.

The topical composition may further comprise a foaming agent as needed. Foaming agents may be quillaia saponaria extract or disodium EDTA (disodium ethylenediaminetetraacetic acid).

Fragrance agents in these embodiments may be added to increase the aesthetic appeal of the topical composition. Fragrances may be propylparaben, hydrogenated castor oil, cinnamal, eugenol, coumarin, linalool, α-isomethyl ionone, hydroxyisohexyl 3-cyclohexene carboxaldehyde, butylphenyl methylpropional, or natural fragrance essential oils.

The topical composition according to the above embodiments may be formulated to be an anti-aging day cream, anti-aging night cream, hand and nail cream, acne treatment cream or tonic, anti-diaper rash cream, shampoo (regular and anti-dandruff), conditioner, face wash, and body wash.

In other embodiments, the topical composition is a lip balm. The topical composition may comprise, apart from CBG hemp oil or CBG cannabis oil at about 1% to 3% by weight of the topical composition, cosmetically suitable waxes, cosmetic oils or butter, flavor, and moisturizing agents. CBG hemp oil or CBG cannabis oil as used in these embodiments may have about 3% to 24% CBG, and 1% to 10% CBD by weight, with other naturally occurring cannabinoids at lower weight percentage.

Suitable waxes for use in this embodiment may be bee wax, lanolin, candelilla, soy, or carnauba wax. Other skin conditioning agents may be added to give the composition a softer profile. Suitable skin conditioning agents may be cocoa butter, *Ricinus communis* (castor) seed oil, olive oil, coconut oil, and avocado oil. Other suitable skin conditioning agents may also be used.

Flavoring oils, such as apple, cherry, green tea, cinnamon, clove, black tea, plum, mango, date, watermelon, coconut, pear, jasmine, peach, fennel, fragrant melon, lychee, mint, chocolate, coffee, cream, banana, almond, grape, strawberry, blueberry, blackberry, pine, kiwi, sapote, taro, lotus, pineapple, orange, lemon, melon, peach, licorice, vanilla, rose, osmanthus, kiwi, ginseng, spearmint, citrus, cucumber, honeydew, walnut, almond, honey, or any suitable flavor, may be added into the topical composition according to this embodiment.

When the topical composition may be a shampoo, conditioner, a face wash, or body wash, the topical composition may contain, apart from CBG hemp oil or CBG cannabis oil, other ingredients common in shampoo, conditioner, face wash, or body wash. As these are rinse-off products, the concentrations of CBG hemp oil or CBG cannabis oil in these products may be at 0.05% to 1% by weight of the topical rinse-off composition.

Suitable surfactants, cleansing agents, additives, antimicrobials, and thickeners in these embodiments may be ammonium chloride, ammonium lauryl sulfate, sodium laureth sulfate, sodium lauryl sulfate, piroctone olamine, sodium lauroamphoacetate, polysorbate 20, polysorbate 80, PEG-150, citric acid, quaternium-15, polyquaternium-10, coco-glucoside, cocamide MEA, methylisothiazolinone, or di-PGG-2 myreth-10 adipate. Piroctone olamine may be used as an antimicrobial agent in shampoo embodiments for anti-dandruff purposes.

In other embodiments, the topical composition may be a conditioner. The conditioner according to embodiments may comprise, in addition to CBD hemp oil or CBG cannabis oil, certain humectants; certain reconstructors such as hydrolyzed protein, glossers such as silicones; thermal protectors; certain lubricants such as fatty alcohols, panthenol, or dimethicone; certain sequestrants, such as sodium gluconate, potassium gluconate, or calcium disodium ethylene diamine tetra-acetate; antistatic agents; or preservatives.

In other embodiments, the topical composition may be a deodorant spray. The deodorant spray according to these embodiments may comprise CBG hemp oil or CBG cannabis oil at 0.1% to 1.5% by weight of the deodorant spray as a whole. CBG hemp oil or CBG cannabis oil as used in these embodiments may have about 3% to 24% CBG, and 1% to 10% CBD by weight, with other naturally occurring cannabinoids at lower weight percentage. The deodorant spray may further comprise alcohol, such as ethanol and isopropyl alcohol as the main carrier. Suitable emulsifiers and humectants may be added, such as glycerin, polysorbate 20, methylcyclodextrin, or ethylhexyl glycerin. Other suitable emulsifiers may also be used. Fragrance in the form of natural essential oils may be added to give a pleasant scent.

Flavoring oils, such as apple, cherry, green tea, cinnamon, clove, black tea, plum, mango, date, watermelon, coconut, pear, jasmine, peach, fennel, fragrant melon, lychee, mint, chocolate, coffee, cream, banana, almond, grape, strawberry, blueberry, blackberry, pine, kiwi, sapote, taro, lotus, pineapple, orange, lemon, melon, peach, licorice, vanilla, rose, osmanthus, kiwi, ginseng, spearmint, citrus, cucumber, honeydew, walnut, almond, honey, or any suitable flavor, may be added into the deodorant spray as fragrance according to these embodiments.

To make the topical composition, ingredients may be obtained according to the embodiments above. Water phase ingredients may be combined and mixed. Oily phase ingredients may be combined and mixed with heat application at about 50 °C to about 70 °C to reduce viscosity and facilitate mixing. Water phase and oil phase may be combined under continuous mixing and heating.

The water phase comprises de-ionized water, bulking agents, cosmetic fillers, emulsifiers, preservatives, and anti-caking agents. Ingredients for the water phase may be combined and mixed. Water phase ingredients may be separated into different sequences as needed.

The oily phase may comprise hemp oil, tocopherol, lipobelle soyaglycone, fragrance, essential oils, and other oily ingredients. Ingredients for the oily phase may be combined and mixed while heated. Other ingredients may be added in sufficient quantities, such as sodium hydroxide for pH balancing purposes.

To make the lip balm composition according to embodiments, ingredients may be obtained according to the above embodiments. In one container, combine waxes and heat to about 50 °C to about 70 °C. In another container, combine skin conditioning oils and hemp oil with CBD and CBG, then heat to about 35 °C to about 45 °C. Combine the oil with the wax; then add flavors as desired. Mix well, pour the hot liquid into molds, then cool down to room temperature. The lip balm composition may be applied to lips.

To make the deodorant spray according to embodiments, ingredients may be obtained according to the description above. A base comprising of water and alcohols may be premixed in a container. Emulsifiers and humectants may be mixed with CBG hemp oil or CBG cannabis oil; then added into the base. Fragrance may be added as needed to the composition.

Anti-aging creams according to embodiments may be applied at least once daily to human skin to reduce signs of aging, including sagging skin, discoloration, dryness, and crow's feet. Hand and nail cream may be applied at least once daily to nourish and moisturize the skin. Acne cream according to the present invention may be applied at least once daily to prevent and treat acne. Lip balm according to these embodiments may be applied as needed to the lips to moisturize and preserve lips' health.

Shampoo, conditioner, face wash, and body wash according to these embodiments may be applied to the hair or skin with water to wash, nourish, and moisturize the hair and the skin. Shampoo, conditioner, face wash, and body wash may be applied daily, or as needed. Deodorant spray may be applied to the skin twice daily or as needed.

### EXAMPLES

### Example 1 (reference)

**Anti-aging day cream with SPF 15**

| **Sequence** | **Ingredient by INCI Name** | **Percentage (wt/wt)** |
|---|---|---|
| 1 | De-ionized water | 47.750 |
| 1 | Disodium EDTA | 0.100 |
| 2 | Acrylate/C 10-30 alkyl acrylate copolymer | 0.200 |
| 1 | Xanthangum | 0.200 |
| 1 | Glycerin | 3.000 |
| 1 | Potassium Cetyl Phosphate | 0.300 |
| 1 | Water | 8.000 |
| | Polyglyceryl-6 Distearate Jojoba Esters | |
| 1 | Polyglyceryl-3 Beeswax, Cetyl Alcohol | 6.000 |
| 1 | Cetearyl Alcohol | 1.500 |
| 2 | Ethylhexyl Methoxycinnamate | 7.000 |
| 2 | Octocrylene | 2.000 |
| 1 | D-Pantenol | 0.500 |
| 2 | Tocopherol | 0.500 |
| 2 | Hempoil (CBD/CBG) | 3.000 |
| 2 | Butyl Methoxydibenzoylmethane | 3.000 |
| 2 | Lipobelle soyaglycone | 2.000 |
| 2 | Coco-Caprylate | 3.000 |
| 2 | Butylene Glycol Cocoate | 3.000 |
| 2 | Prophylheptyl Caprylate | 4.000 |
| 1 | Preservative | 1.000 |
| 3 | Water, Sodium Hydroxide | 0.450 |
| | Aluminum Starch | |
| 1 | Octenylsuccinate | 3.000 |
| 2 | Fragrance | 0.500 |
| | **Total** | **100.00** |

### Formulation procedure:

1. In a beaker, combine Sequence #1 ingredients, mix and set aside.
2. In another beaker, combine Sequence #2 ingredients, mix while heat to 50 °C for at least 15 minutes.
3. Heat the beaker with Sequence #1 to 60 °C, add Sequence #2 to Sequence #1, continue to mix and heat until the beaker reaches 60 °C. Continue to mix for another 10 minutes.
4. Cool the beaker content down to room temperature.
5. Adjust the batch to pH 6.8 - 8 with Sequence #3.

### Example 2

**Anti-aging night cream**

| **Sequence** | **Ingredient by INCI Name** | **Percentage (wt/wt)** |
|---|---|---|
| 1 | De-ionized Water | 63.2700 |
| 2 | Butyrospermum Parkii | 6.3500 |
| 2 | Butylene Glycol Dicaprylate/dicaprate | 5.0000 |
| 1 | Hydrogenated Lecithin | 4.5000 |
| 1 | Glycerin | 3.1875 |
| 1 | Betaine | 3.0000 |
| 2 | Pentylene Glycol | 2.2500 |
| 1 | Xylitylglucoside | 1.5000 |
| 1 | Anhydroxyxylitol | 0.9000 |
| 2 | Fragrance | 0.7500 |
| 2 | Lupinus Albas Seed Oil | 0.7000 |
| 1 | Xylitol | 0.6000 |
| 2 | Phenoxyethanol | 0.5175 |
| 1 | Sodium Gluconate | 0.5000 |
| 1 | Ethylhexylglycerin | 0.5000 |
| 2 | Squalene | 0.4500 |
| 2 | Hempoil (CBD/CBG) | 2.0000 |
| 1 | Acrylates/C 10-30 Alkyl Acrylate Crosspolymer | 0.3000 |
| 2 | Lipobelle soyaglycone | 3.0000 |
| 2 | Triticum Vulgare (Wheat) Germ Oil Unsaponifiables | 0.3000 |
| 1 | Alkyl Methacrylates Crosspolymer | 0.2100 |
| 3 | Potassium Hydroxide | 0.1000 |
| 1 | Sodium Carboxymethyl Betaglucan | 0.0700 |
| 1 | Imidazolidinyl Urea | 0.0175 |
| 1 | Sodium Chloride | 0.0165 |

### Formulation procedure:

1. In a beaker, combine Sequence #1 ingredients, mix and set aside.
2. In another beaker, combine Sequence #2 ingredients, mix while heat to 50 °C for at least 15 minutes.
3. Heat the beaker with Sequence #1 to 60 °C, add Sequence #2 to Sequence #1, continue to mix and heat until the beaker reaches 60 °C. Continue to mix for another 10 minutes.
4. Cool the beaker content down to room temperature.
5. Adjust the batch to pH 6.8 - 8 with Sequence #3.

### Example 3 (reference)

**Lip balm**

| **Sequence** | **Ingredient by INCI Name** | **Percentage (wt/wt)** |
|---|---|---|
| 2 | Cocoa Butter | 25.00 |
| 1 | Beeswax | 21.00 |
| 1 | Lanolin | 18.00 |
| 1 | Carnauba wax | 22.00 |
| 2 | Avocado oil | 5.00 |
| 2 | Hemp oil (CBD/CBD) | 2.00 |
| 3 | Flavor | 6.00 |
| 2 | Tocopherol | 1.00 |
| | **Total** | **100.00** |

### Formulation procedure:

1. Combine Sequence #1 in the main beaker, heat to 65 °C and mix. Cool sequence #1 to around 35 °C.
2. In another beaker, combine Sequence #2, heat to 40 °C and mix. Pour Sequence #2 into Sequence #1.
3. Add Sequence #3 into the main beaker and mix. Pour the mix into cylindrical molds. Cool the mixture down to room temperature.

### Example 4

**Eye cream**

| **Sequence** | **Ingredient by INCI name** | **Percentage (wt/wt)** |
|---|---|---|
| 1 | De-ionized Water | 59.8800 |
| 2 | Cetearyl Alcohol | 7.5000 |
| 2 | Butylene Glycol Dicaprylate/dicaprate | 5.0000 |
| 2 | Dicaprylyl Carbonate | 6.0000 |
| 1 | Betaine | 3.0000 |
| 1 | Cyathea Cumingii Leaf Extract | 3.0000 |
| 1 | Glycerin | 2.0000 |
| 2 | Behenyl Alcohol | 1.8000 |
| 2 | Glyceryl Stearate | 1.4000 |
| 2 | Butylene Glycol | 1.0000 |
| 1 | Lecithin | 0.9600 |
| 1 | Phenoxyethanol | 0.5000 |
| 2 | Ethylhexylglycerin | 0.5000 |
| 2 | Fragrance | 0.6000 |
| 1 | Sodium Gluconate | 0.2500 |
| 1 | Carnosine | 1.0000 |
| 2 | Propylene Glycol | 0.0500 |
| 2 | Glycine Soja (Soybean) Sterols | 1.0000 |
| 1 | Lactic Acid | 0.0280 |
| 1 | Butylphenyl Methylpropional | 0.0280 |
| 1 | Kigelia Africana Extract | 0.0500 |
| 3 | Quillaia Saponaria Extract | 0.0500 |
| 2 | Hydroxyisohexyl 3-Cyclohexene Carboxaldehyde | 0.0200 |
| 2 | Hemp oil (CBD/CBG) | 2.0000 |
| 2 | Coumarin | 0.0180 |
| 2 | Linalool | 0.0103 |
| 2 | Alpha-Isomethyl Ionone | 0.0056 |
| 2 | Lipobelle soyaglycone | 2.0000 |
| 2 | Tocopherol | 0.1000 |
| 2 | Eugenol | 0.1000 |
| 2 | Cinnamal | 0.0500 |
| 1 | Silybum Marianum/Silybum Marianum Fruit Extract | 0.1000 |

### Example 5 (reference)

**Hand and nail cream**

| **Sequence** | **Ingredient by INCI Name** | **Percentage (wt)/wt)** |
|---|---|---|
| 1 | Aqua | 56.42 |
| 1 | Ethylhexyl Stearate | 4.00 |
| 1 | Ethylhexyl Methoxycinnamate | 5.00 |
| 1 | Polyglyceryl-3 Methylglucose Distearate | 5.00 |
| 2 | Dicaprylyl Carbonate | 4.00 |
| 1 | Glycerin | 6.00 |
| 1 | Biosaccharide Gum-1 | 2.00 |
| 2 | Paraffinum Liquidum | 4.00 |
| 2 | Isopropyl Palmitate | 2.00 |
| 2 | Glyceryl Stearate | 3.00 |
| 2 | Hempoil (CBD/CBG) | 2.00 |
| 1 | Cetearyl Alcohol | 2.00 |
| 1 | Vitis Vinifera | 1.00 |
| 1 | D-Panthenol | 1.00 |
| 2 | Fragrance | 0.60 |
| 1 | Hydrolyzed Keratin | 0.50 |
| 1 | Phenoxyethanol | 0.12 |
| 2 | Allantoin | 0.50 |
| 2 | Methylparaben | 0.10 |
| 1 | Lactic Acid | 0.08 |
| 2 | PEG-40 Hydrogenated Castor Oil | 0.10 |
| 1 | Propylparaben | 0.05 |
| 1 | Ethylhexyl glycerin | 0.03 |
| 2 | Tocopherol | 0.50 |
| | **Total** | **100.00** |

### Formulation procedure:

1. In a beaker, combine Sequence #1 ingredients, mix and set aside.
2. In another beaker, combine Sequence #2 ingredients, mix while heat to 50 °C for at least 15 minutes.
3. Heat the beaker with Sequence #1 to 60 °C, add Sequence #2 to Sequence #1, continue to mix and heat until the beaker reaches 60 °C. Continue to mix for another 10 minutes.
4. Cool the beaker content down to room temperature.

The following examples are provided as examples of some embodiments of this invention, without limiting the invention in anyway.

### Example 6 (reference)

**Diaper rash cream**

| **Ingredient by INCI Name** | **Percentage** |
|---|---|
| De-ionized water | 68.50 |
| Polyglyceryl-3 Methylglucose Distearate | 5.00 |
| Ethylhexyl Stearate | 3.00 |
| Glycerin | 5.00 |
| Cetyl Alcohol | 4.00 |
| Oxidized Corn Oil | 4.00 |
| Butylene Glycol Dicaprylate/dicaprate | 4.00 |
| Hempoil (CBD/CBG) | 1.00 |
| Decyl Oleate | 2.00 |
| Phenoxyethanol | 0.50 |
| Ethylhexylglycerin | 0.50 |
| Polyacrylamide | 1.00 |
| Fragrance | 0.60 |
| C13-14 Isoparrafin | 0.40 |
| Allantoin | 0.30 |
| Laureth-7 | 0.10 |
| Lactic Acid | 0.10 |
| **Total** | **100.00** |

### Example 7 (reference)

**Anti-acne tonic**

| **Ingredient by INCI Name** | **Percentage** |
|---|---|
| De-ionized Water | 55.40 |
| Alcohol | 31.00 |
| Sucrose Laurate | 1.50 |
| Diglyceryn | 2.00 |
| Glucose | 2.00 |
| Methyl Gluceth-10 | 1.50 |
| Glyceryl Caprylate | 1.50 |
| PPG-26-Buteth-26 | 1.00 |
| Hempoil (CBD/CBG) | 1.00 |
| Panthenol | 0.70 |
| Phenoxyethanol | 0.50 |
| Ethylhexyl glycerin | 0.50 |
| Isopropyl Alcohol | 0.50 |
| Fragrance | 0.50 |
| Allantoin | 0.20 |
| Potassium Ascorbyl Tocopheryl Phosphate | 0.10 |
| Lactic Acid | 0.10 |
| **Total** | **100.00** |

### Example 8

**Anti-acne cream**

| **Ingredient by INCI Name** | **Percentage** |
|---|---|
| De-ionized Water | 58.800 |
| Cetearyl Alcohol | 8.000 |
| Dicaprylyl Carbonate | 7.000 |
| Glycerin | 6.000 |
| Butylene Glycol Dicaprylate/dicaprate | 4.000 |
| Oxidized corn oil | 2.000 |
| Caprylic/Capric Triglyceride | 2.500 |
| Polyacrylate 13 | 2.000 |
| Glyceryl Caprylate | 1.000 |
| Cetearyl Glucoside | 0.900 |
| Polyisobutene | 1.000 |
| Hempoil (CBD/CBD) | 2.000 |
| Sodium Gluconate | 1.000 |
| Fragrance | 0.600 |
| Polysorbate 20 | 0.200 |
| Sodium Hydroxide | 0.200 |
| Sodium Chloride | 0.100 |
| Allantoin | 0.500 |
| Oleic Triglyceride | 0.200 |
| Sodium Stearoyl Lactylate | 0.100 |
| Tocopheryl Acetate | 0.500 |
| Sodium Lactate | 0.100 |
| Cetyl Alcohol | 0.100 |
| Sodium Carboxymethyl Betaglucan | 0.100 |
| Phytosterol | 0.100 |
| Carnosine | 0.002 |
| Phenoxyethanol | 0.500 |
| Ethylhexyl glycerin | 0.500 |
| Citric Acid | 0.002 |
| **Total** | **100.0** |

### Example 9 (reference)

**Face wash formulation**

| **Ingredient by INCI Name** | **Percentage** |
|---|---|
| De-ionized Water | 51.10 |
| Alcohol | 10.00 |
| Glycerin | 12.00 |
| Cocamidopropyl Betaine | 10.00 |
| Decyl Glucoside | 6.00 |
| PEG-200 Hydrogenated Glyceryl Palmate | 3.00 |
| PEG-7 Glyceryl Cocoate | 2.00 |
| Methyl Gluceth-10 | 2.00 |
| Acrylates/C 10-30 Alkyl Acrylate Crosspolymer | 2.00 |
| Cellulose Gum | 0.50 |
| Potassium Hydroxide | 0.20 |
| Hemp oil (CBD/CBG) | 0.20 |
| Phenoxyethanol | 0.50 |
| Ethylhexyl glycerin | 0.50 |
| **Total** | **100.00** |

### Example 10 (reference)

**Body wash formulation**

| **Ingredient by INCI Name** | **Percentage** |
|---|---|
| De-ionized Water | 70.04 |
| Sodium Laureth Sulfate | 6.00 |
| Glycerin | 3.50 |
| Cocamidopropyl Betaine | 10.00 |
| Coco-Glucoside | 2.00 |
| Glyceryl Oleate | 2.00 |
| Sodium Chloride | 1.00 |
| Propylene Glycol | 1.00 |
| Fragrance | 1.00 |
| Glycol Distearate | 1.00 |
| Ammonium Lauryl Sulfate | 0.50 |
| Cocamide MEA | 0.20 |
| Laureth-10 | 0.20 |
| Guar Hydroxypropyltrimonium Chloride | 0.10 |
| PEG-200 Hydrogenated Glyceryl Palmate | 0.10 |
| Sodium Hydroxide | 0.05 |
| PEG-7 Glyceryl Cocoate | 0.10 |
| Lactic Acid | 0.01 |
| Hemp oil (CBD/CBG) | 0.20 |
| Phenoxyethanol | 0.50 |
| Ethylhexyl glycerin | 0.50 |
| **Total** | **100.00** |

### Example 11 (reference)

**Anti-dandruff shampoo**

| **Ingredient by INCI Name** | **Percentage** |
|---|---|
| De-ionized Water | 49.92 |
| Glycerin | 15.00 |
| Cocamidopropyl Betaine | 20.00 |
| Methyl Gluceth-10 | 4.00 |
| Sodium Chloride | 2.00 |
| PEG-800 | 3.00 |
| Hemp oil (CBD/CBG) | 0.20 |
| Glyceryl Oleate | 1.00 |
| Piroctone olamine | 0.30 |
| Panthenol | 1.00 |
| Coco-Glucoside | 1.00 |
| Cellulose Gum | 0.40 |
| Sodium Gluconate | 0.50 |
| Fragrance | 0.60 |
| Citric Acid | 0.08 |
| Phenoxyethanol | 0.50 |
| Ethylhexyl glycerin | 0.50 |
| **Total** | **100.00** |

### Example 12 (reference)

**Deodorant spray**

| **Ingredient by INCI Name** | **Percentage** |
|---|---|
| Ethanol | 72.50 |
| De-ionized Water | 14.00 |
| Glycerin | 6.00 |
| Isopropyl Alcohol | 3.00 |
| Fragrance | 3.00 |
| Ethylhexylglycerin | 1.00 |
| Hemp oil (CBD/CBG) | 0.50 |
| **Total** | **100.00** |

## Claims

1. A topical composition for dermal application comprising:
hemp oil extractable from the *Cannabis sativa* plant containing cannabigerol at 3 to 24 by weight percent and cannabidiol at 1 to 10 by weight percent;
at least one anti-oxidant selected from the group consisting of *Vitis vinifera* extract, tocopherols, β-carotene, anthocyans, catechins, quercetins, genistein extract, acetyl hexapeptide-3, *Silybum marianum* fruit extract, and silymarin;
at least one anti-microbial agent selected from a group consisting of imidazolidinyl urea, sodium chloride, *Matricaria chamomilla* extract. *Aloe vera* extract. *Calendula officinalis* extract, *Kigelia africana* extract, *Lavandulla officinallis* essential oil, *Melaleuca alternifolia* essential oil, piroctone olamine, and *Cinnamomum zeylanicum* essential oil;
at least one anti-inflammatory agent selected from the group consisting of curcumin extract, evodia extract, *Camellia sinesis* extract, cocoa seed butter, citric acid, salicylic acid, tocopherol, loganin, palmatine, and *Vitis vinifera* extract;
at least one preservative;
at least one emulsifier;
suitable cosmetic additives and carriers; and
de-ionized water.

2. The topical composition of Claim 1, wherein hemp oil comprises 0.2% to 10% by weight of the topical composition.

3. The topical composition of Claim 1, wherein hemp oil comprises 0.05% to 1% by weight of the topical composition.

4. The topical composition of Claim 1, further comprising at least one anti-glycation agent selected from the group consisting of extracts of *Vaccinium angustifolium,* ergothioneine, hydroxystilbenes, benfotimaine, pyrisoxamine, G-rutin, pyridoxal phosphate, green tea extract, curcumin extract, *Lupius albas* seed oil, and *Kigelia africana* extract.

5. The topical composition of Claim 4, further comprising at least one UV ray filter selected from the group consisting of butylene glycol dicaprylyate, butylene glycol dicaprate, ethylhexyl methoxycinnamate, bis-ethylhexylloxyphenol methoxyphenyl triazine, camphor benzalkonium methosulfate, diethylhexyl butamido triazone, bisdisulizole disodium, drometrizole trisiloxane, ethylhexyl triazone, methyl anthranilate, 4-methylbenzylidene camphor, methylene bis-benzotriazolyl tetramethylbutyl phenol, octocrylene, butyl methoxydibenzoylmethane, phenylbenzimidazole sulfonic acid, polyacrylamidomethyl benzylidene camphor, and terephthalylidene dicamphor sulfonic acid.

6. The topical composition of Claim 5, further comprising at least one skin conditioning agent selected from the group consisting of butyrospermum parkii, pentylene glycol, acetyl hexapeptide-3, lipobelle soyaglycone, xylitylglucoside, anhydroxyxylitol, xylitol, ethylhexylglycerin, squalene, *Triticum vulgare* germ oil unsaponifiables, ceramide-3, ethylhexyl stearate, biosaccharide gum-1, paraffinum liquidum, isopropyl palminate, glycerine stearate, D-panthenol, lactic acid, tocopherols, cetearyl alcohol, dicaprylyl carbonate, butylene alcohol, butylene glycol, carnosine, butylene glycol cocoate, propylheptyl caprylate, glycine soja sterols, decyl oleate, PPG-26-buteth-26, potassium ascorbyl tocopheryl phosphate, guar hydroxypropyltrimonium chloride, methyl gluceth-10, and avocado oil.

7. The topical composition of Claim 6, further comprising a soothing agent.

8. The topical composition of Claim 7, further comprising at least one keratolytic agent selected from the group consisting of hydrolyzed keratin and allantoin.

9. The topical composition of any of Claims 1-8, wherein the topical composition is a day cream, a night cream, an eye cream, a hand and nail cream, anti-diaper rash cream, a shampoo, a conditioner, a body wash, a face wash, an acne treatment cream, or an acne treatment tonic.

10. The topical composition of any of Claims 1-8, wherein the topical composition is an acne treatment cream, or an acne treatment tonic.

11. Topical composition comprising hemp oil comprising cannabigerol and cannabidiol according to any one of Claims 1-8 for use in the therapeutic treatment of skin conditions.

12. Topical composition comprising hemp oil comprising cannabigerol and cannabidiol according to any one of Claims 1-8 for use in the therapeutic treatment of acne.

## Patentansprüche

1. Eine topische Zusammensetzung zur dermalen Anwendung, die umfasst:
Hanföl, das aus der *Cannabis sativa-Pflanze* extrahierbar ist, enthaltend Cannabigerol mit 3 bis 24 Gew.-% und Cannabidiol mit 1 bis 10 Gew.-%;
zumindest ein Antioxidationsmittel, ausgewählt aus der Gruppe, bestehend aus *Vitis vinifera*-Extrakt, Tocopherolen, β-Carotin, Anthocyanen, Katechinen, Quercetinen, Genisteinextrakt, Acetylhexapeptid-3, *Silybum marianum-*Fruchtextrakt und Silymarin;
zumindest ein antimikrobielles Mittel, ausgewählt aus der Gruppe, bestehend aus Imidazolidinylharnstoff, Natriumchlorid, *Matricaria chamomilla-Extrakt, Aloe vera*-Extrakt, *Calendula officinalis-Extrakt, Kigelia africana*-Extrakt, *Lavandulla officinallis*-ätherisches Öl, *Melaleuca alternifolia*-ätherisches Öl, Pirocton-Olamin und *Cinnamomum zeylanicum*-ätherisches Öl;
zumindest ein entzündungshemmendes Mittel, aus gewählt aus der Gruppe, bestehend aus Curcumin-Extrakt, Evodia-Extrakt, *Camellia sinesis*-Extrakt, Kakaosamenbutter, Zitronensäure, Salicylsäure, Tocopherol, Loganin, Palmitin und *Vitis vinifera-*Extrakt;
zumindest ein Konservierungsmittel;
zumindest einen Emulgator;
geeignete kosmetische Zusätze und Trägerstoffe; und
entionisiertes Wasser.

2. Die topische Zusammensetzung nach Anspruch 1, wobei das Hanföl 0,2 bis 10 Gew.-% der topischen Zusammensetzung ausmacht.

3. Die topische Zusammensetzung nach Anspruch 1, wobei Hanföl 0,05 bis 1 Gew.-% der topischen Zusammensetzung ausmacht.

4. Die topische Zusammensetzung nach Anspruch 1, ferner umfassend zumindest ein Antiglykierungsmittel, das ausgewählt ist aus der Gruppe bestehend aus Extrakten von *Vaccinium angustifolium,* Ergothionein, Hydroxystilbenen, Benfotiamin, Pyrisoxamin, G-Rutin, Pyridoxalphosphat, Grünteeextrakt, Curcumin-Extrakt, *Lupius albas*-Samenöl und *Kigelia africana*-Extrakt.

5. Die topische Zusammensetzung nach Anspruch 4, ferner umfassend zumindest einen UV-Strahlenfilter, ausgewählt aus der Gruppe, bestehend aus Butylenglykoldicaprylyat, Butylenglykoldicaprat, Ethylhexylmethoxycinnamat, bis-Ethylhexylloxyphenolmethoxyphenyltriazin, Kampferbenzalkoniummethosulfat, Diethylhexylbutamidtriazon, Bisdisulizoldinatrium, Drometrizoltrisiloxan, Ethylhexyltriazon, Methylanthranilat, 4-Methylbenzylidenkampfer, Methylen-bisbenzotriazolyltetramethylbutylphenol, Octocrylen, Butylmethoxydibenzoylmethan, Phenylbenzimidazolsulfonsäure, Polyacrylamidomethylbenzylidenkampfer und Terephthalylidendikampfersulfonsäure.

6. Die topische Verbindung nach Anspruch 5, ferner umfassend zumindest ein Hautkonditionierungsmittel, ausgewählt aus der Gruppe, bestehend aus Butyrospermum parkii, Pentylenglykol, Acetylhexapeptid-3, Lipobelle-Soyaglycon, Xylitylglucosid, Anhydroxyxylitol, Xylitol, Ethylhexylglycerin, Squalen, *Triticum vulgare*-Keimöl unverseifbar, Ceramid-3, Ethylhexylstearat, Biosaccharid-Gummi-1, flüssiges Paraffin, Isopropylpalminat, Glycerinstearat, D-Panthenol, Milchsäure, Tocoperhole, Cetearylalkohol, Dicaprylylcarbonat, Butylenalkohol, Butylenglykol, Carnosin, Butylenglykolcocoat, Propylheptylcaprylat, Glycinsojasterole, Decyloleat, PPG-26-Buteth-26, Kaliumascorbyltocoperhylphosphat, Guarhydroxypropyltrimoniumchlorid, Methylgluceth-10 und Avocadoöl.

7. Die topische Zusammensetzung nach Anspruch 6, ferner umfassend ein Beruhigungsmittel.

8. Die topische Zusammensetzung nach Anspruch 7, ferner umfassend zumindest ein keratolytisches Mittel, ausgewählt aus hydrolysiertem Keratin und Allantoin.

9. Die topische Zusammensetzung nach einem der Ansprüche 1 bis 8, wobei die topische Zusammensetzung eine Tagescreme, eine Nachtcreme, eine Augencreme, eine Hand- und Nagelcreme, eine Creme gegen Windelausschlag, ein Shampoo, eine Spülung, ein Duschgel, ein Gesichtswasser, eine Aknebehandlungscreme oder ein Aknebehandlungstonikum ist.

10. Topische Zusammensetzung nach einem der Ansprüche 1 bis 8, wobei die topische Zusammensetzung eine Aknebehandlungscreme oder ein Aknebehandlungstonikum ist.

11. Topische Zusammensetzung, umfassend Hanföl, umfassend Cannabigerol und Cannabidiol nach einem der Ansprüche 1 bis 8 zur Verwendung in der therapeutischen Behandlung von Hautproblemen.

12. Topische Zusammensetzung, umfassend Hanföl, umfassend Cannabigerol und Cannabidiol nach einem der Ansprüche 1 bis 8 zur Verwendung in der therapeutischen Behandlung von Akne.

## Revendications

1. Composition topique pour application dermique comprenant :
de l'huile de chanvre extractible de la plante *Cannabis sativa* contenant du cannabigérol à 3 à 24 pour cent en poids et du cannabidiol à 1 à 10 pour cent en poids ;
au moins un anti-oxydant choisi dans le groupe consistant en un extrait de *Vitis vinifera,* les tocophérols, le β-carotène, les anthocyanes, les catéchines, les quercétines, un extrait de génistéine, l'acétyl hexapeptide-3, un extrait de fruit de *Silybum marianum* et la silymarine ;
au moins un agent antimicrobien choisi dans un groupe consistant en l'imidazolidinyl urée, le chlorure de sodium, un extrait de *Matricaria chamomilla,* un extrait d'*Aloe vera,* un extrait de *Calendula officinalis,* un extrait de *Kigelia africana,* l'huile essentielle de *Lavandulla officinallis,* l'huile essentielle de *Melaleuca alternifolia,* la piroctone olamine et l'huile essentielle de *Cinnamomum zeylanicum ;*
au moins un agent anti-inflammatoire choisi dans le groupe consistant en un extrait de curcumine, un extrait d'evodia, un extrait de *Camellia sinesis,* le beurre de fèves de cacao, l'acide citrique, l'acide salicylique, le tocophérol, la loganine, la palmatine et un extrait de *Vitis vinifera ;*
au moins un conservateur ;
au moins un émulsifiant ;
des additifs et supports cosmétiques appropriés ; et
de l'eau désionisée.

2. Composition topique selon la revendication 1, dans laquelle l'huile de chanvre représente 0,2 % à 10 % en poids de la composition topique.

3. Composition topique selon la revendication 1, dans laquelle l'huile de chanvre représente 0,05 % à 1 % en poids de la composition topique.

4. Composition topique selon la revendication 1, comprenant en outre au moins un agent anti-glycation choisi dans le groupe consistant en les extraits de *Vaccinium angustifolium,* l'ergothionéine, les hydroxystilbènes, la benfotiamine, la pyridoxamine, la G-rutine, le phosphate de pyridoxal, un extrait de thé vert, un extrait de curcumine, de l'huile de graines de *Lupinus albus* et un extrait de *Kigelia africana.*

5. Composition topique selon la revendication 4, comprenant en outre au moins un filtre à rayons UV choisi dans le groupe consistant en le dicaprylate de butylène glycol, le dicaprate de butylène glycol, le méthoxycinnamate d'éthylhexyle, la bis-éthylhexyloxyphénol méthoxyphényl triazine, le camphre benzalkonium méthosulfate, la diéthylhexyl butamido triazone, le bisdisulizole disodique, le drométrizole trisiloxane, l'éthylhexyl triazone, l'anthranilate de méthyle, le 4-méthylbenzylidène camphre, le méthylène bis-benzotriazolyl tétraméthylbutyl phénol, l'octocrylène, le butyl méthoxydibenzoylméthane, l'acide phénylbenzimidazole sulfonique, le polyacrylamidométhyl benzylidène camphre et l'acide téréphtalylidène dicamphre sulfonique.

6. Composition topique selon la revendication 5, comprenant en outre au moins un agent de conditionnement de la peau choisi dans le groupe consistant en le butyrospermum parkii, le pentylène glycol, l'acétyl hexapeptide-3, la lipobelle soyaglycone, le xylitylglucoside, l'anhydroxyxylitol, le xylitol, l'éthylhexylglycérol, le squalène, les insaponifiables d'huile de germe de *Triticum vulgare,* le céramide-3, le stéarate d'éthylhexyle, la gomme biosaccharide-1, le paraffinum liquidum, le palminate d'isopropyle, le stéarate de glycérine, le D-panthénol, l'acide lactique, les tocophérols, l'alcool cétéarylique, le carbonate de dicaprylyle, le butylène alcool, le butylène glycol, la carnosine, le cocoate de butylène glycol, le caprylate de propylheptyle, les glycine soja stérols, l'oléate de décyle, le PPG-26-buteth-26, le potassium ascorbyl tocophéryl phosphate, le chlorure de guar hydroxypropyltrimonium, le méthyl gluceth-10 et l'huile d'avocat.

7. Composition topique selon la revendication 6, comprenant en outre un agent apaisant.

8. Composition topique selon la revendication 7, comprenant en outre au moins un agent kératolytique choisi dans le groupe consistant en la kératine hydrolysée et l'allantoïne.

9. Composition topique selon l'une quelconque des revendications 1 à 8, dans laquelle la composition topique est une crème de jour, une crème de nuit, une crème pour les yeux, une crème pour les mains et les ongles, une crème anti-érythème fessier, un shampooing, un conditionneur, un nettoyant pour le corps, un nettoyant pour le visage, une crème de traitement contre l'acné ou un traitement tonique contre l'acné.

10. Composition topique selon l'une quelconque des revendications 1 à 8, dans laquelle la composition topique est une crème de traitement contre l'acné ou un tonique pour le traitement de l'acné.

11. Composition topique comprenant de l'huile de chanvre contenant du cannabigérol et du cannabidiol selon l'une quelconque des revendications 1 à 8 pour une utilisation dans le traitement thérapeutique d'affections cutanées.

12. Composition topique comprenant de l'huile de chanvre contenant du cannabigérol et du cannabidiol selon l'une quelconque des revendications 1 à 8 pour une utilisation dans le traitement thérapeutique de l'acné.
